Europäisches Patentamt

**European Patent Office**

**Office européen des brevets**

(19)

(11) Publication number: **0 010 738**
**A1**

# EUROPEAN PATENT APPLICATION

(12)

(21) Application number: **79104144.5**

(22) Date of filing: **25.10.79**

(51) Int. Cl.³: **A 61 K 39/00**

(30) Priority: **25.10.78 DK 4752/78**

(43) Date of publication of application: **14.05.80**
**Bulletin 80/10**

(84) Designated Contracting States: **AT BE CH DE FR GB IT**
**LU NL SE**

(71) Applicant: **A/S Alfred Benzon, Halmtorvet 29, DK-1700**
**Copenhagen V (DK)**

(72) Inventor: **Osther, Kurt Bakgaard, Lyngbakkevej 49B,**
**DK-2850 Narum (DK)**

(74) Representative: **Patentanwälte Dipl.-Ing. A. Grünecker,**
**Dr.-Ing. H. Kinkeldey, Dr.-Ing. W. Stockmair,, Dr. rer.**
**nat. K. Schumann, Dipl.-Ing. P.H. Jakob, Dr. rer. nat. G.**
**Bezold Maximilianstrasse 43, D-8000 München 22 (DE)**

(54) Transfer factor and method for producing same.

(57) Transfer Factor to be used therapeutically by paren-
teral administration in human beings to obtain immunity
directed against an antigen which is pathogenic in man,
such as cancer cells or extracts thereof, is produced by
immunizing pigs or other large non-primate mammal host
animals with the antigen and thereafter obtaining Transfer
Factor from leukocytes from the animal. The separation of
Transfer Factor from the leukocytes is performed by
incubating the leukocytes and thereafter rupturing them,
preferably lyophilizing the ruptured cell material, and per-
forming dialysis to exclude molecules having a molecular
weight of above 10,000.

EP 0010738 A1

0010738

Transfer Factor and Method for Producing Same.

The present invention relates to a method for producing Transfer Factor to be used therapeutically by injection into human beings to obtain an immunity directed against an antigen which is pathogenic in man.

It is well-known to use Transfer Factors specific to various antigens which are pathogenic in man in the therapy of the corresponding diseases.

The rationale in the use of Transfer Factor as anti-tumor therapy is that individuals immunized with tumor antigens will passively (through contamination) or actively (through parenteral administration) give rise to production of Transfer Factor directed against tumor antigens (Vetto, R.M., Burger, D.R., Nolte, J.E. & Vandenbark, A.A., Transfer Factor Immunotherapy in Cancer, 1977).

Transfer Factor may induce "delayed hypersensitivity" in individuals in which it is administered parenterally (Welch, T.M., Wilson, G.B. & Fudenberg, H.H., Human Transfer Factor in Guinea Pigs: Further Studies. In: The Mediators of Cellular Immunity (G.D. Novelli, ed.) 1977). Thus, dialysed Transfer Factor from leukocytes of human origin may transfer local and systemical cellular immunity and cutaneous "delayed hypersensitivity" to non-immunized individuals (Wilson, G.B., Welch, T.M. & Fudenberg, H.H., Tx: A Component in Human Dialyzable Transfer Factor that Induces Cutaneous Delayed Hypersensitivity in Guinea Pigs, Clin. Immunol. Immunopathol. 7:187-202, 1977). The literature concerning the effect of Transfer Factor on various cell types is scarce, but it is presumed that lymfocytes and monocytes are involved in the active immuno-process induced by Transfer Factor (Mitchison, N.A. & Waksman, B.H., In: Basis of Induction in Cellular Immunity (H.S. Lawrence & M. Landy, eds.) Academic Press, New York, 1969, page 719; Nelson, D.S., In: Macrophages and Cellular Immunity (A. Laskin & H. Lechevalier, ed.), Butterworths, London, 1972, page 45).

The activity mechanism of Transfer Factor is described in Hugh Fudenberg's work concerning characterization of the "target cell"

2  0010738

for Transfer Factor (Arala-Chaves, M.P., Silva, A., Porto, M.T.R., Ramos, M.T.F. & Fudenberg, H.H., In Vitro Characterization of the Target Cell for Transfer Factor-Dialyzable Leucocyte Extracts, In: The Target Cell for Transfer Factor, 1977).

Among scientific investigations concerning the treatment of patients suffering from cancer is noted a work by Levin, Fudenberg et al. relating to tumor-specific Transfer Factor of human origin used for the treatment of patients with osteogenic sarcoma by repeated subcutaneous injections (Levin, A.S., Byers, V.S., Fudenberg, H.H., Johnston, J.O. & Spitler, L.E., Immunologic Parameters before and during Immunotherapy with Tumour-Specific Transfer Factor, J.Clin. Invest. 55, 487-499, 1975) later followed up in a long term study: Byers, V.S., LeCam, L., Levin, A.S., Johnston, J.O and Hackett, A.J., Immunotherapy of Osteogenic Sarcoma with Transfer Factor, Long-Term Follow-Up Cancer Immunol. Immunotherapy 6, 243-253, 1979.

Specific Transfer Factor is a dialyzable product contained in dialysates from white blood cells originating from a host organism which is immunized with a homologous specific antigen.

The activity of Transfer Factor is not species specific, which is primarily due to is low molecular weight (Boucheix, C.L., Phillips, J., Pizza, G., Sartorio, C. & Viza, D., Activity of Animal Transfer Factor in Man. The Lancet, i:1977).

The method of the invention for producing Transfer Factor to be used therapeutically by injection into human beings to obtain immunity directed against antigen which is pathogenic in man comprises immunizing a non-primate host animal with the antigen and thereafter obtaining therapeutically useful Transfer Factor from leukocytes from the animal. The principle of the invention is that antigens which are pathogenic in man are used for immunizing non-primate host animals, in particular pigs or other large mammals. This produces a Transfer Factor of the animal leukocytes directed against the antigen in question and useful in human therapy. The Transfer

3  0010738

Factor may be separated by dialysing the white blood cells of the host animal.

In the present context, the term "antigen which is pathogenic in man" is intended to designate a protein or a proteinaceous substance which is capable of eliciting an immune response in the host animal to result in a production of Transfer Factor transferring immunity against the said antigen.

As examples of antigens which are pathogenic in man may be mentioned cancer cells and extracts thereof, infectious fungi, infectious bacteria, infectious vira, and infectious rickettsia.

The invention is of particular importance in connection with the production of Transfer Factor directed against human cancer (tumor), but is also generally applicable for the treatment of diseases where the patient suffers from immunodefects such as patients under immunosuppressive treatment or patients during cytostatic treatment, for example, patients suffering from leukaemia.

One major advantage of producing Transfer Factor by immunization of pigs or other non-primates is that pigs or other non-primates can be immunized, without any ethical restrictions, with antigens which are pathogenic in man. There would be ethical objections against actively immunizing human beings with various pathogenic antigens such as cancer cells or extracts thereof. An advantage of using pigs as Transfer Factor-producers is that the pig is immunologically closely related to primates. However, because of the low molecular weight of Transfer Factor of about 5000, it must be presumed that also other non-primate host animals such as horse, cow and sheep would be useful as host animals for producing Transfer Factor with specificity against antigens which are pathogenic in man, including cancer cells in vitro and in vivo, and with tolerability in human beings, considering that the risk that such heterologous Transfer Factor should be capable of causing an immunization of human beings against the Transfer Factor itself on parenteral administration will be small due to the low molecular weight of the Transfer

4     **0010738**

Factor. This also conforms with the fact that it is generally known to use heterologous insulin (pigs and cows) for human application, even though the molecular weight of insulin is higher than the molecular weight of Transfer Factor and that it is generally known that heterologous ACTH (adreno-corticotropic hormone) having a molecular weight of about the same order of size as Transfer Factor, is well tolerated by human beings.

The porcine Transfer Factor or Transfer Factor from other animal species is dialyzable. This offers the advantage that by dialyzing a Transfer Factor-containing protein suspension, one obtains, in the dialysis liquid, exclusively Transfer Factor and contaminating molecules having a molecular size determined by the porosity of the dialysis tube. Hence, for the production of Transfer Factor, one chooses a dialysis tube which allows passage of molecules of the size less than 6000-10.000 or any other form of separation of molecules, for example gel filtration, ultrafiltration or ultracentrifugation, allowing separation of molecules having a molecular weight below 6000-10.000. Also, Amicon filters or membranes such as TC5E and PM 10 membrane may be used. Furthermore, it is possible to utilize separation aiming at obtaining molecules with a molecular weight below 6000-10.000 by means of a vacuum dialysis apparatus. Even though the separation, in principle, aims at only allowing passage of molecules having a molecular weight below 6000, in other words, in the lower end of the above-stated interval, it is often preferred to use methods which allow passage of molecules having a somewhat higher molecular weight, that is, up to 10.000, in other words, the upper end of the above-stated interval, as this results in an optimum obtainment of molecules having a molecular weight in the range of about 5000. Furthermore, column chromatography separation methods as described by G. David Novelli in The Mediators of Cellular Immunity 1977 may be used. Other methods for separating molecules with different molecular weight or charge such as electrofocussing or ion exchange may, of course, also be used for obtaining Transfer Factor from the non-human leukocytes.

As mentioned above, Transfer Factor is generally useful in the treatment of various human diseases such as immune defects, virus diseases or bacterial diseases and certain cancer diseases. Especially should be noted Allan S. Levin et al.'s immune therapy of osteogenic sarcoma using human tumor-specific Transfer Factor. As donors for Transfer Factor in that article, human beings were used which were or have been in close contact with the patient treated with this Transfer Factor (A.S. Levin, V.S. Byers, H. Hugh Fudenberg, J. Wybran, A.J. Hackett, J.O. Johnson and L.E. Spitler, J.Clin.Invest. 55:487-499, 1975).

One therapeutic advantage in producing cancer-specific porcine or other large mammal Transfer Factor upon immunization of the host animal with the human cancer cells in question is that it becomes possible to produce Transfer Factor against various cancer types by active immunization of the host animal with these various cancer cell types. In each single case, this results in a Transfer Factor which is useful for the treatment of patients suffering from the same type of cancer. In preliminary experiments, it has been found that a cancer-specific Transfer Factor produced from leukocytes from pigs immunized with human cancer cells of osteogenic sarcoma type, is tolerated by the human patient during a long term treatment (injection of porcine Transfer Factor).

Furthermore, it has been found that the effect on the cancer disease of the patient upon instituted therapy with porcine Transfer Factor was the following: increase in the number of white blood cells, increase in the hemoglobin percentage, increase in the thrombocyte count, decrease in basic phosphatases (basic phosphatases are increased at osteogenic sarcoma) to the normal range. Furthermore, technetium scanning (isotope scanning) showed a considerable reduction of the tumor size. It should be noted that cytostatic treatment with methotrexate was given three weeks prior to the institution of the Transfer Factor treatment without any reduction of the tumor size; it is known that methotrexate exerts its activity within three weeks from instituted treatment.

6 **0010738**

As mentioned above, porcine Transfer Factor with cancer specificity has been obtained from white blood cells derived from a pig immunized with human cancer cells. However, as mentioned above, it is also possible to use other antigens which are pathogenic in man for immunizing the host animal in order to harvest Transfer Factor inducing the corresponding immunities, for example, antigen pertaining to fungus infections, bacterial infections and virus infections, rickettsia infections and any other infection.

The term "cancer (tumor)" used above is intended to designate cancer cells generally, cancer cells pertaining to tumors and also tumor cells which are not necessarily of malignant character.

Example.

Human cancer cells (osteogenic sarcoma cells) were obtained during operation and by biopsy of cancer patients. The cancer cells were immediately transported in Eagle MEM, optionally admixed with foetal calf serum or any other nutrient substrate, to the laboratory, where the cell material was seeded in monolayer cultures and in suspension cultures. After colonisation, the cancer culture was again seeded in three passages, until a homogeneous culture has been obtained. The cell culture was marked with the cancer type.

After a sufficient growth of the cell culture, the cells were trypsinized and frozen and thawn twice (until the cell membranes were ruptured).

The cell culture treated in this manner was admixed with equal parts (volume/volume) of Freund's Complete Adjuvant, and the admixture was used for immunization as described below.

Also centrifugation (1800 rpm/15 minutes/4C) of the cell residues has been employed, whereafter the supernatant together with an equal portion (volume/volume) of Freund's Complete Adjuvant was used for the immunization described below.

For the immunization, pigs of an age of about 2 months were used. The immunization was performed by injection in subcutis on the neck in total 5 times with intervals of a fortnight.

After the fifth immunization, the pig was bled. The blood was kept liquid with citrate (ACD) as anticoagulation agent. The leukocytes were separated, and the rest of contaminating red blood cells was removed from the leukocyte suspension by repeated washing with cold 0.83% ammonium chloride solution. Leukocyte count (counting of buffy coats) was performed. About 200 ml buffy coat were obtained per pig, and to this was added cold, sterile 0.83% ammonium chloride solution corresponding to 10%. The mixture was allowed to stand in a refrigerator for 10 minutes. The hemolyzed buffy coat was centrifuged in cooling centrifuge at 800 rpm/25 minutes. The cells were washed in cold $K_2$EDTA-PBS. Thereafter, careful mixing of buffy coats and renewed hemolyzation with cold, sterile ammonium chloride solution were performed. The resulting material was allowed to stand for 10 minutes in refrigerator and was thereafter centrifuged in cooling centrifuge at 800 rpm/25 minutes.

The leukocyte mixture was stirred in an ice bath during the leukocyte count. Eagle MEM with human serum or foetal calf serum was added until the final concentration of leukocytes was $6.6 \times 10^7$ cells per ml.

The leukocyte suspension was incubated at 37.5°C for 4 hours under stirring.

After the incubation was finished, the leukocyte suspension was frozen to -40°C and thawed and frozen twice, until the cells were ruptured.

The volume of the frozen cell material was 320 ml. The cell count was $6.6 \times 10^7$.

8        0010738

$320 \text{ ml} \times 6.6 \times 10^7 \text{ leukocytes} =$
$2.1 \times 10^{10} \text{ leukocytes} =$
$21 \times 10^9 \text{ leukocytes}$

1 Transfer Factor unit (TFA unit) = $1 \times 10^9$ leukocytes.

The cell material was thawed in a water bath and distributed into sterile vials, frozen and lyophilized. Subsequent to lyophilization, 32 ml glass-distilled water were added (10 times concentration in relation to the above-mentioned 320 ml).

The 32 ml of redissolved cell material were poured into a dialysis tube with exclusion limit at the molecular weight 10.000. The tube was tied up and immersed in cold, sterile glass-distilled water (450 ml). Dialysation was performed at 4°C for 2 days.

The dialysis tube with content was discarded. The dialysis liquid which now contained Transfer Factor, was distributed in vials and deep-frozen. Thereafter, lyophilization was performed. The lyophilized amount was measured out in such a way that each vial contained 0.3 TFA-unit.

For administration, the freeze-dried Transfer Factor is redissolved in glass-distilled water and injected subcutaneously or intramuscularly in the patient. The injections are repeated a suitable number of times until the desired tumor reduction has been obtained, or until no further effect can be shown. The above-mentioned effect on a case of osteogenic sarcoma was obtained by injection of 1 Transfer Factor unit per week.

**0010738**

Claims.

1. A method for producing Transfer Factor to be used therapeutically by parenteral administration into human beings to obtain immunity directed against an antigen which is pathogenic in man, c h a r a c t e r i z e d  by immunizing a non-primate host animal with the antigen and thereafter obtaining therapeutically useful Transfer Factor from leukocytes from the animal.

2. A method according to claim 1, c h a r a c t e r i z e d  in that the host animal is a pig or another large mammal.

3. A method as claimed in claim 1 or 2, c h a r a c t e r i z e d  in that the host animal is immunized with human cancer cells or extracts thereof as the antigen which is pathogenic in man.

4. A method according to any of the preceding claims, c h a r a c t e r i z e d  in that Transfer Factor is isolated from the leukocytes from the immunized animal by means of a molecular size separation method which allows passage of molecules having a molecular weight of below 6000-10.000, preferably a method which allows passage of molecules having a molecular weight below 10.000.

5. A method according to claim 4, c h a r a c t e r i z e d  in that the molecular weight separation method is dialysis.

6. A method as claimed in claim 4 or 5, c h a r a c t e r i z e d  in that prior to the separation of Transfer Factor, the leukocytes are incubated and thereafter ruptured.

7. A method as claimed in claim 6, c h a r a c t e r i z e d  in that the incubated and ruptured cell material is lyophilized prior to dialysis.

8. Porcine Transfer Factor which transfers immunity directed against an antigen which is pathogenic in man.

9. Porcine Transfer Factor as claimed in claim 8 which transfers immunity directed against a human cancer type.

| Category | Citation of document with indication, where appropriate, of relevant passages | Relevant to claim |
|---|---|---|
| XP | US - A - 4 132 776 (WAYBURNS-JETER) <br> * Column 1, line 52 to column 2, line 21 * | 1 |
| | FR - A - 2 164 900 (HARTENBACH WALTER) <br> * The whole document * | 1-3 |
| | US - A - 3 991 182 (LYNN E. SPITLER et al. ) <br> * Column 3, line 45 to column 4, line 12 * | 4-7 |
| A | DE - A - 1 617 865 (THEURER KARL) <br> * The whole document * | 1 |

**DOCUMENTS CONSIDERED TO BE RELEVANT**

**CLASSIFICATION OF THE APPLICATION (Int. Cl. 3)**

A 61 K 39/00

**TECHNICAL FIELDS SEARCHED (Int.Cl. 3)**

A 61 K 39/00
A 61 K 37/14

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 11-02-1980 | REMPP |

EPO Form 1503.1   06.78